# EUROPEAN PATENT SPECIFICATION

(11) **EP 3 137 679 B1**
(45) Date of publication and mention of the grant of the patent: **12.06.2019**
(21) Application number: 15721286.1
(22) Date of filing: 28.04.2015
(51) Int. Cl.: D21H 11/18, D21H 17/25

(54) **METHOD FOR PRODUCING A SUSPENSION OF MICROFIBRILLATED CELLULOSE, MICROFIBRILLATED CELLULOSE AND ITS USE**
VERFAHREN ZUR HERSTELLUNG VON MIKROFIBRILLIERTEN CELLULOSE, MIKROFIBRILLIERTE CELLULOSE UND IHRE VERWENDUNG
PROCEDE POUR LA FABRICATION DE LA CELLULOSE MIKROFIBRILLÉE, CELLULOSE MIKROFIBRILLÉE AINSI QUE SA UTILISATION

(30) Priority: 28.04.2014 FI 20145390
(43) Date of publication of application: 08.03.2017
(73) Proprietor: Kemira Oyj, 00180 Helsinki (FI)
(72) Inventor: LILLANDT, Marcus, FI-10210 Ingå (FI); LUNDIN, Tom, FI-20540 Åbo (FI)
(74) Representative: Berggren Oy, Turku
(86) International application number: PCT/FI2015/050289
(87) International publication number: WO 2015/166141

(56) References cited:
- WO-A1-2007/001229
- WO-A1-2009/126106
- WO-A1-2010/092239
- US-A1- 2005 272 836

## Description

The present invention relates to a method for producing a suspension of microfibrillated cellulose according to the preambles of the enclosed claims.

Microfibrillated cellulose (MFC) is produced from various fibre sources comprising cellulosic structures, such as wood pulp, sugar beet, bagasse, hemp, flax, cotton, abaca, jute, kapok and silk floss. Microfibrillated cellulose comprises liberated semi-crystalline nano-sized cellulose fibrils having high length to width ratio. A typical nano-sized cellulose fibril has a width of 5 - 60 nm and a length in a range from tens of nanometres to several micrometres.

Microfibrillated cellulose is produced by using high-pressure homogenizers or fluidizers, in a process where the cell walls of cellulose containing fibres are delaminated and the nano-sized cellulose fibrils are liberated. The process is extremely energy intensive, which increases the production costs of microfibrillated cellulose. Furthermore, homogenizers and fluidizers are easily clogged by the natural fibres comprising cellulosic structures. In order to minimise these drawbacks the natural fibres are pre-treated before they are homogenized, e.g. by using various mechanical/enzymatic treatments, oxidation, introduction of charges through carboxymethylation, etc. Production of microfibrillated cellulose is discussed, for example, in Ankerfors, M., "Microfibrillated cellulose: Energy efficient preparation techniques and key properties", Licentiate Thesis, KTH Royal Institute of Technology, Stockholm, Sweden, 2012.

The interest for microfibrillated cellulose has increased during the last years, as the material has shown promising potential in a variety of applications, for example in food processing or for use in food products, pharmaceuticals or advanced materials, which comprise of metallic, ceramic, polymer, cementitious and wood materials and various compositions of these materials. Consequently there is a need for effective and economical methods for producing microfibrillated cellulose.

WO 2010/092239 discloses a method for producing modified nanofibrillated cellulose. In the method cellulosic material is brought into a fibre suspension, a cellulose derivative or polysaccharide is adsorbed onto fibres in said suspension under special conditions and the obtained fibre suspension derivative is subjected to mechanical disintegration, whereby modified nanofibrillated cellulose is obtained. The obtained modified nanofibrillated cellulose comprises the cellulose derivative or polysaccharide which was adsorbed onto fibres, and the adsorbed modifies cannot be separated from the obtained product.

US 2005/0272836 discloses water-dispersible cellulose and its production process. In the process an aqueous dispersion of cellulose fibrous particles are subjected to fibre-shortening and micronizing and the aqueous dispersion is treated by a high-pressure homogenizer.

WO 2007/001229 discloses a method for preparing microfibrillar polysaccharide, where the method comprises treating of polysaccharide in an aqueous suspension comprising an oxidant and at least one transition metal and mechanically delaminating the polysaccharide.

WO 2009/126106 discloses a method for providing a nanocellulose involving modifying cellulose fibres. The method includes a first modification of the cellulose material, where the fibres are treated with an aqueous electrolyte-containing solution of amphoteric cellulose derivative, thereafter the modification is followed by a mechanical treatment.

An object of this invention is to minimise or possibly even eliminate the disadvantages existing in the prior art.

An object of the present invention is to provide a simple method for producing microfibrillated cellulose, which can be easily purified.

A further object of the invention is to provide pure nanocellulose, which is free of process modifiers.

These objects are attained with a method and an arrangement having the characteristics presented below in the characterising parts of the independent claims.

A typical method for producing a suspension of microfibrillated cellulose is defined by claim 1 and comprises at least the following steps:
- obtaining an aqueous suspension of natural cellulose fibres,
- adding an additive consisting of at least one natural polymer to the suspension of natural cellulose fibres,
- feeding the obtained mixture comprising natural cellulose fibres and the additive into a homogenizer or a fluidizer, and
- obtaining the suspension of microfibrillated cellulose.

Microfibrillated cellulose is obtained by using the method according to the present invention.

Typical use of microfibrillated cellulose is for oil drilling and mining applications, for food manufacture, in food products, cosmetics and/or pharmaceuticals.

Typically microfibrillated cellulose is used for rheology control, structural applications and/or for manufacture of pulp and paper products.

Now it has been surprisingly found out that it is possible to produce microfibrillated cellulose in a homogenizer or a fluidizer without clogging problems by simply adding an additive consisting of at least one natural polymer to the aqueous suspension of natural cellulose fibres before the suspension is fed into the homogenizer or fluidizer. No pre-treatment of the natural cellulose fibres is necessary, which makes the process effective and economical, also in large industrial scale. Furthermore, the natural polymer is not irrevocably bound or adsorbed to the cellulose fibres or to the produced microfibrillated cellulose. This means that the natural polymer can be removed from the produced microfibrillated cellulose for example by washing. Still further, as the process also employs only aqueous solutions without chemical additives, e.g. organic solvents, the produced microfibrillated cellulose is suitable for uses demanding high purity, e.g. in production of food products or pharmaceuticals. The present invention thus provides a simple method for producing pure microfibrillated cellulose in a cost effective manner.

In context of the present application the term "natural cellulose fibres" denotes cellulose fibres that originate from seed plant material, i.e. gymnosperm and angiosperm plant material, such as wood, sugar beets, bagasse, potatoes, carrots, sisal, hemp, flax, abaca, jute, kapok, cotton or wheat straw. The natural cellulose fibres are manufactured by using conventional pulping processes. The cellulose fibres may be, if desired, washed, bleached and/or dried before they are used for production of microfibrillated cellulose by homogenization or fluidization, but they are otherwise chemically, enzymatically and mechanically unrefined, untreated, unhydrolyzed, un-oxidized, unconditioned, ungrafted and/or unmodified after the production of cellulose fibre pulp. For example, fluff pulp fibres are excluded from the natural cellulose fibres.

According to one preferred embodiment of the invention the aqueous suspension of natural cellulose fibres comprises mainly water as the liquid phase. The liquid phase of the aqueous suspension comprises > 70 weight-%, preferably > 85 weight-% of water, the water content typically being in the range of 70 - 100 weight-%, more typically 85 - 100 weight-%, even more typically 90 - 100 weight-%, sometimes even 97 - 100 weight-%, of the liquid phase. Preferably the aqueous suspension of natural cellulose fibres is free from organic liquids. According to one embodiment the aqueous suspension of natural cellulose fibres is obtained by suspending the natural cellulose fibres in water.

Microfibrillated cellulose is used synonymously with terms "cellulose microfibrils", "microfibrillar cellulose", and "nanofibrillated cellulose". In the context of the present application the term "microfibrillated cellulose" is understood as liberated semi-crystalline cellulosic fibril structures or as liberated bundles of nano-sized cellulose fibrils. Microfibrillated cellulose has a diameter of 2 - 60 nm, preferably 4 - 50 nm, more preferably 5 - 40 nm, and a length of several micrometers, preferably less than 500 µm, more preferably 2 - 200 µm, even more preferably 10 - 100 µm, most preferably 10 - 60 µm. Microfibrillated cellulose comprises often bundles of 10 - 50 microfibrils. Microfibrillated cellulose may have high degree of crystallinity and high degree of polymerization, for example the degree of polymerisation DP, i.e. the number of monomeric units in a polymer, may be 100 - 3000. Further, microfibrillated cellulose may have as a suspension a high elastic modulus, for example in the range of 10 - 10⁵ Pa.

According to one preferred embodiment the natural cellulose fibres originating from hardwood are used for producing the suspension of microfibrillated cellulose. The natural cellulose fibres may be bleached or unbleached. The natural cellulose fibres may be selected from birch fibres, eucalyptus fibres, acacia fibres, aspen fibres, maple fibres, poplar fibres, locust fibres or any mixture thereof. According to one especially preferred embodiment the natural cellulose fibres are bleached birch fibres.

The additive, which is added to the suspension of natural cellulose fibres before homogenization or fluidization, comprises at least one natural polymer. The term "natural polymer" is here understood as a polymeric material or compound which originates from non-petroleum material occurring originally in nature. The at least one natural polymer in the additive is selected from group consisting of carboxymethyl cellulose (CMC), methyl cellulose, hydroxypropyl cellulose, starch, carrageenan, locust bean gum, tamarind gum, chitosan, chitin, guar gum, cellulosic derivatives, such as nanofibrillated cellulose, and any of their mixtures. According to one preferred embodiment the additive consists of natural polymer which is starch and/or carboxymethyl cellulose. Preferably the natural polymer in the additive is carboxymethyl cellulose. The natural polymer, which is used as additive, is preferably water-soluble and it may be cationic, anionic or amphoteric. According to an embodiment of the invention the natural polymer in the used additive is cationic starch.

The additive may comprise two or more different natural polymers. In case two or more natural polymers are used, they may be added to the suspension of natural cellulose fibres separately but simultaneously, or they may be intermixed with each other to form a single additive, which is added to the suspension of natural cellulose fibres.

The natural polymer may be added in amount of 2 - 75 weight-%, preferably 5 - 60 weight-%, more preferably 7 - 50 weight-%, even more preferably 10 - 30 weight-%, calculated from weight of the total dry solid content of the suspension of natural cellulose fibres. According to one preferable embodiment the additive is added in such amount that the natural polymer(s) may be added in amount of 15 - 75 weight-%, preferably 17 - 60 weight-%, more preferably 20 - 50 weight-%, even more preferably 23 - 30 weight-%, calculated from weight of the total dry solid content of the suspension of natural cellulose fibres.

According to the invention, the additive consists solely of one or more natural polymers, without any other chemicals. The additive is free from any electrolytes comprising monovalent and/or polyvalent cations.

According to one preferred embodiment of the invention the additive consisting of at least one natural polymer is added to the suspension of natural cellulose fibres at temperature of < 160 °C, preferably < 80 °C, more preferably < 60 °C, even more preferably < 30 °C. The temperature may be during the addition in the range of 5 - 160 °C or 5 - 80 °C, preferably 10 - 60 °C, more preferably 15 - 35 °C, even more preferably 15 - 30 °C. Thus no heating of the cellulose fibre suspension is necessary, which reduces the energy consumption of the process and make it easier to perform also in a large scale.

The time between the addition of the additive to the suspension of natural cellulose fibres and the feeding of the mixture of natural cellulose fibres and additive into the homogenizer or fluidizer may be < 1500 min, preferably < 30 min, more preferably < 15 min, even more preferably < 5 min.

There is no adsorption of the additive's natural polymer onto the natural cellulose fibres or any permanent attachment between the natural cellulose fibre and the natural polymer. This means that no specific reaction time is necessary between the addition of the additive to the natural cellulose fibre suspension and the processing of the mixture in the homogenizer or fluidizer. According to the invention the mixture of natural cellulose fibres and the additive is fed immediately and directly into the homogenizer or fluidizer after the addition of the additive to the suspension of the natural cellulose fibres.

The mixture of natural cellulose fibres and the additive may be fed into the homogenizer or fluidizer at feed consistency of 1 - 50 weight-%, preferably 1 - 30 weight-%, more preferably 2 - 20 weight-%, even more preferably 3 - 15 weight-%, sometimes even 5 - 15 weight-%, calculated as dry solids. The high feed consistency enables the production of redispersible microfibrillated cellulose with high consistency, which reduces the need for drying of the microfibrillated cellulose after its production by homogenization or fluidization. The microfibrillated cellulose produced in this manner is dispersible into water and has good usability in various applications described below.

All conventional homogenizers and fluidizers available may be used, such as Gaulin homogenizer or microfluidizer. The homogenization or fluidization may be performed under the influence of a pressure difference. During homogenization or fluidization the mixture comprising natural cellulose fibres is subjected to high pressure of 500 - 2100 bar. For example, in homogenization the mixture comprising natural cellulose fibres and the additive may be pumped at high pressure, as defined above, and fed through a spring-loaded valve assembly. The natural cellulose fibers in the mixture are subjected to a large pressure drop under high shearing forces. This leads to fibrillation of the natural cellulose fibers. Alternatively, in fluidization homogenization the mixture comprising natural cellulose fibres and the additive passes through Z-shaped channels under high pressure, as defined above. The channel diameter may be 200 - 400 µm. Shear rate, which is applied to the natural cellulose fibres in the mixture is thus high, and results in the formation of cellulose microfibrils. Irrespective of the procedure, i.e. homogenization or fluidization, which is used for producing the microfibrillated cellulose, the procedure may be repeated several passes until the desired degree of fibrillation is obtained.

The produced microfibrillated cellulose may have solids content in the range of 1 - 50 weight-%, preferably 1 - 30 weight-%, more preferably 2 - 20 weight-%, even more preferably 3 - 15 weight-%, sometimes even 5 - 15 weight-%, calculated as dry solids. The microfibrillated cellulose obtained is in form of fibrils, suspension or a stable gel. The microfibrillated cellulose is free of organic liquids, i.e. organic solvents.

The produced microfibrillated cellulose comprises adsorbed inorganic electrolytes preferably less than 4 mg/g dry microfibrillated cellulose, more preferably 2 mg/g dry microfibrillated cellulose. The electrolyte amounts are determined from the microfibrillated cellulose directly and immediately after its production, without any intermediate washing steps between the production and determination. This means that it is possible to produce microfibrillated cellulose that comprises minimal amounts of inorganic cations, such as calcium.

According to one embodiment of the invention the additive, i.e. the natural polymer(s), is removed from the produced suspension of microfibrillated cellulose. The removal may be done, for example, by washing with water. In this manner it is possible to obtain microfibrillated cellulose that is suitable even to uses with high purity demands.

Microfibrillated cellulose, which is produced by using the method described, may be used, for example, as a viscosity modifier in oil drilling and mining applications. Furthermore it may be used in production of food products, cosmetics and/or pharmaceuticals as an interfacial agent/additive, a surface active agent/additive, a release agent/additive, a vehicle agent/additive or structural agent/additive. It may be used for dispersion or suspension control, as dispersing, stabilizing or rheology agent. It may be used as a part of single-, two- or multicomponent fluid rheology agent. For example, it may be used for rheology control, structural applications and/or for manufacture of pulp and paper products. It may also be used for manufacturing of solids structures, such as transparent films, or as non-caloric food additive.

According to one preferred embodiment the microfibrillated cellulose, which is produced by using the method described, is used in production of pulp, paper and/or board as a filler, strength additive, coating or barrier agent. According to one embodiment of the invention the microfibrillated cellulose is used for production of the outer or inner layer(s) of multilayered boards.

### EXPERIMENTAL

Some embodiments of the invention are described more closely in the following non-limiting examples.

### Example 1

Homogenisation of four different suspension samples were performed in order to produce microfibrillated cellulose.

Commercial birch kraft pulp was used in Sample 2, 3 and 4, and carboxymethyl cellulose, CMC, Finnfix 300 supplied by CP Kelco, was used in Samples 1, 2 and 4.
Sample 1 comprised microcrystalline cellulose, MCC, and CMC, in ratio 1:1, dry solids content of the suspension was 1.5 weight-%.
Sample 2 comprised birch kraft pulp and CMC, in ratio 1:1, dry solids content of the suspension was 1.5 weight-%.
Sample 3 comprised 100 % birch kraft pulp, dry solids content of the suspension was 0.7 weight-%.
Sample 4 comprised birch kraft pulp and CMC, in ratio 1:1, dry solids content of the suspension was 1.4 weight-%

Samples were dispersed in water using an Ultraturrax. Thereafter the samples were homogenized in Ariete NS3006 homogenizer at 1000 bar.

The fibrillation of the samples was characterised by light transmittance at wavelength 800 nm, which is known to correlate with the changes in degree of fibrillation. The light transmittance was measured with a Perkin Elmer Lambda 900 UVA/IS/NIR spectrophotometer from a homogenised sample diluted to 0.1 weight-% for Samples 1, 2 and 3, and to 0.2 weight-% for Sample 4. The results are shown in Table 1. The transmittance wavelengths 400 nm, 600 nm, 800 nm and 1000 nm were compared.

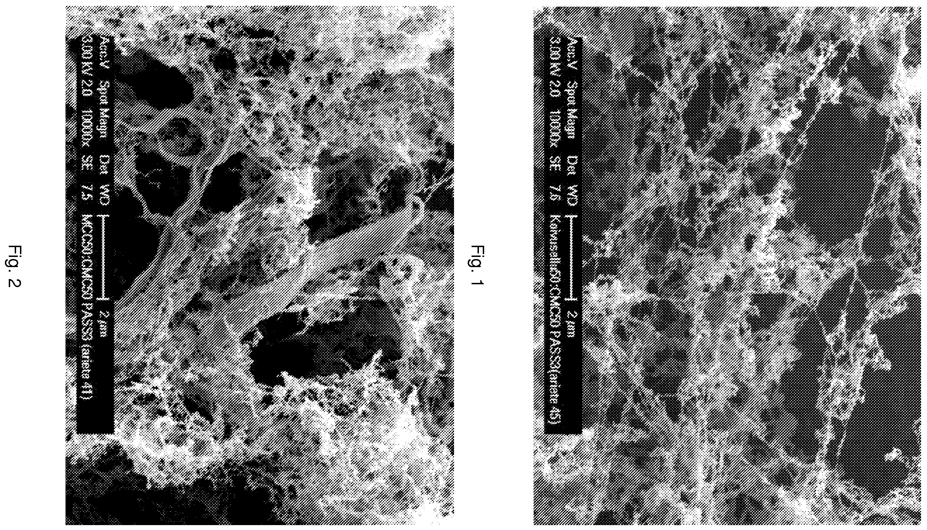

The decline in light transmittance after the first pass is due to formation of larger fibrils and release of initial fines. Beyond two passes the transmittance values were stabilised or slightly increased, indicating formation of microfibrillated cellulose. From Table 1 it can be seen that Samples 2 and 4 resulted in a significantly higher transmittances after two passes, when compared to Sample 1. This indicates a better fibrillation of Samples 2 and 4. These results are also confirmed by data in Figures 1 and 2. Figure 1 represents an electron microscopy figure of Sample 2 after 3 passes, with a high degree of fibrillation. Shown in Figure 2 is an electron microscopy figure of Sample 1 after 3 passes. It is apparent that the degree of the fibrillation in Figure 2 (Sample 1) was smaller than in Figure 1(Sample 2).

From the transmittance data in Table 1 it is apparent that no significant fibrillation of the birch pulp occurred at 0.7 weight-% without CMC addition in Sample 3.

### Example 2

A calibration curve was prepared by preparing aqueous solutions comprising different amounts of carboxymethyl cellulose (CMC) and measuring the charge (µeq/l) of the solution as a function of CMC concentration (g/l).

The reference sample was prepared by first washing a pulp sample with deionised water. Thereafter a slurry with pulp consistency of 30 g/l containing 0.05 M CaCl₂ and 0.01 M NaHCO₃ was prepared and heated to 75 - 80 °C. 20 mg carboxymethyl cellulose was added per gram of pulp (o.d.). The pH was adjusted to pH 7.5 - 8 with 1 M NaOH. The slurry was mixed for 2 h at 75 - 80 °C, and homogenized in a fluidizer. Slurry with 2 % consistency was obtained.

Sample according to the invention was prepared by using a pulp slurry with same consistency as the reference sample. Same amount of carboxymethyl cellulose (CMC) as in reference sample was added to the slurry at room temperature just before homogenization. Slurry with 2 % consistency was obtained.

After homogenization the obtained nanocellulose slurry samples were either filtered or centrifuged. Charge of the liquid phase was determined, and the amount of released CMC was estimated on basis of the calibration curve. The results are shown in Table 2.

The percentage values for samples according to invention are >100 % because some charges are released from the fibres from the homogenization. However, it can be seen from Table 2 that in practice all CMC is removed from the fibres during. In reference samples about 75 % of CMC remains adsorbed onto the fibres.

**Table 1. Light transmittance data of different suspension samples, indicating the degree of fibrillation in the sample.**

| | | **Transmittance, %** | | | |
|---|---|---|---|---|---|
| | | **wavelength, nm** | | | |
| **Sample** | **weight-%** | **400** | **600** | **800** | **1000** |
| **No 1** | 1.5 | 20.5445 | 27.864 | 34.163 | 39.8385 |
| PASS 1 | 1.5 | 11.6975 | 18.4105 | 24.269 | 29.181 |
| PASS 2 | 1.5 | 7.148 | 12.8125 | 18.571 | 24.2815 |
| PASS 3 | 1.5 | 6.5435 | 12.268 | 18.2895 | 23.851 |
| PASS 5 | 1.5 | 6.8605 | 13.414 | 20.598 | 27.684 |
| **No 2** | 1.5 | 52.2645 | 57.754 | 60.3255 | 61.9025 |
| PASS 1 | 1.5 | 48.8625 | 52.5195 | 56.707 | 54.9245 |
| PASS 2 | 1.5 | 41.7765 | 44.445 | 47.718 | 50.0245 |
| PASS 3 | 1.5 | 42.625 | 47.995 | 52.4135 | 53.296 |
| PASS 5 | 1.5 | 42.8625 | 51.993 | 56.0035 | 58.6085 |
| **No 3** | 0.7 | 39.279 | 40.95 | 42.782 | 42.751 |
| PASS 1 | 0.7 | 34.149 | 38.214 | 40.516 | 42.048 |
| PASS 2 | 0.7 | 27.458 | 32.354 | 35.095 | 36.756 |
| PASS 3 | 0.7 | 24.965 | 30.451 | 33.573 | 35.499 |
| PASS 5 | 0.7 | 15.223 | 23.607 | 29.405 | 33.539 |
| **No 4** | 1.4 | 60.719 | 64.135 | 62.044 | 64.436 |
| PASS 1 | 1.4 | 50.961 | 54.961 | 59.14 | 61.855 |
| PASS 2 | 1.4 | 48.372 | 53.71 | 57.194 | 60.272 |
| PASS 3 | 1.4 | 50.492 | 56.33 | 58.119 | 59.304 |
| PASS 5 | 1.4 | 33.662 | 43.437 | 49.341 | 52.505 |

**Table. 2 Results of Example 2**

| **Sample** | **Sample dewatering** | **Consistency [%]** | **CMC added [g/L]** | **CaCl₂ [M]** | **NaHCO₃ [M]** | **Charge [µeq/L]** | **Removed CMC [%]** |
|---|---|---|---|---|---|---|---|
| Invention (fibre + CMC) | centrifuged | 2 | 1 | - | - | -3961.1 | 106 |
| Invention (fibre + CMC) | filtrated | 2 | 1 | - | - | -3831.6 | 103 |
| Reference | centrifuged | 2 | 1 | 0.05 | 0.01 | -1412.7 | 38 |
| Reference | filtrated | 2 | 1 | 0.05 | 0.01 | -1310.9 | 35 |

## Claims

1. Method for producing a suspension of microfibrillated cellulose having a diameter of 2 - 60 nm and a length less than 500 µm, comprising at least the following steps:
- obtaining an aqueous suspension of natural cellulose fibres,
- adding to the suspension of natural cellulose fibres an additive, which consists of at least one natural polymer selected from carboxymethyl cellulose (CMC), methyl cellulose, hydroxypropyl cellulose, starch, carrageenan, locust bean gum, tamarind gum, chitosan, chitin, guar gum, cellulosic derivatives, such as nanofibrillated cellulose, and any of their mixtures,
- feeding the obtained mixture consisting of natural cellulose fibres and the additive directly into a homogenizer or a fluidizer after the addition of the additive to the suspension of natural cellulose fibres, and
- obtaining a suspension of microfibrillated cellulose.

2. Method according to claim 1, **characterized in** adding the additive in such amount that the natural polymer(s) are added to the aqueous suspension of natural cellulose fibres in amount of 2 - 75 weight-%, preferably 5 - 60 weight-%, more preferably 7 - 50 weight-%, even more preferably 10 - 30 weight-%, calculated from weight of the total dry solid content.

3. Method according to claim 1, **characterized in** adding the additive in such amount that the natural polymer(s) are added to the aqueous suspension of natural cellulose fibres in amount of 15 - 75 weight-%, preferably 17 - 60 weight-%, more preferably 20 - 50 weight-%, even more preferably 23 - 30 weight-%, calculated from weight of the total dry solid content of the suspension of natural cellulose fibres.

4. Method according to any of claims 1 to 3, **characterized in that** the additive may comprise two or more different natural polymers

5. Method according to claim 1, **characterized in that** the additive comprises starch and/or carboxymethyl cellulose.

6. Method according to any of preceding claims 1 to 5, **characterized in that** the additive is removed from the produced suspension of microfibrillated cellulose.

7. Method according to any of claims 1 to 6, **characterized in** feeding the mixture of natural cellulose fibres and the additive into the homogenizer or fluidizer at feed consistency of 1 - 50 weight-%, preferably 1 - 30 weight-%, more preferably 2 - 20 weight-%, even more preferably 3 - 15 weight-%, calculated as dry solids

8. Method according to any of claims 1 to 7, **characterized in that** the natural cellulose fibres originate from hardwood.

9. Method according to claim 8, **characterized in that** the natural cellulose fibres are bleached or unbleached and selected from birch fibres, eucalyptus fibres, acacia fibres, aspen fibres, poplar fibres, locust fibres, maple fibres or a mixture thereof, preferably bleached birch fibres, .

10. Method according to any of claims 1 to 9, **characterized in** adding the additive to the suspension of natural cellulose fibres at temperature of < 160 °C, preferably < 80 °C, more preferably < 60 °C, even more preferably in the range of 15 - 35 °C.

11. Method according to any of claims 1 to 10, **characterized in that** the homogenization or fluidization is performed under the influence of a pressure difference.

12. Method according to any of claims 1 to 11, **characterized in that** the aqueous suspension of natural cellulose fibres is free from organic liquids.

13. Method according to claim 1, **characterised in that** the obtained microfibrillated cellulose has solids content in the range of 1 - 50 weight-%, preferably 1 - 30 weight-%, more preferably 2 - 20 weight-%, even more preferably 3 - 15 weight-%, calculated as dry solids, and that it is in form of a stable gel.

## Patentansprüche

1. Verfahren zur Herstellung einer Suspension von mikrofibrillierter Cellulose mit einem Durchmesser von 2-60 nm und einer Länge von weniger als 500 µm, umfassend mindestens die nachstehenden Schritte:
- Erhalten einer wässrigen Suspension von natürlichen Cellulosefasern,
- Zugabe eines Additivs, welches aus mindestens einem natürlichen Polymer besteht, ausgewählt aus Carboxymethylcellulose (CMC), Methylcellulose, Hydroxypropylcellulose, Stärke, Carrageen, Johannisbrotkernmehl, Tamarindgummi, Chitosan, Chitin, Guarkernmehl, Cellulosederivaten, wie nanofibrillierte Cellulose, und einem ihrer Gemische, zu der Suspension von natürlichen Cellulosefasern,
- direktes Einspeisen des aus natürlichen Cellulosefasern bestehenden erhaltenen Gemisches und dem Additiv in einen Homogenisator oder einen Fluidisator nach der Zugabe des Additivs zu der Suspension von natürlichen Cellulosefasern, und
- Erhalten einer Suspension von mikrofibrillierter Cellulose.

2. Verfahren gemäß Anspruch 1, **gekennzeichnet durch** die Zugabe des Additivs in einer derartigen Menge, dass die/das natürliche Polymere(n) zu der wässrigen Suspension von natürlichen Cellulosefasern in einer Menge von 2-75 Gew.-%, vorzugsweise 5-60 Gew.-%, besonders bevorzugt 7-50 Gew.-% und insbesondere 10-30 Gew.-% zugegeben werden, berechnet aus dem Gewicht des Gesamttrockenfeststoffgehalts.

3. Verfahren gemäß Anspruch 1, **gekennzeichnet durch** die Zugabe des Additivs in einer solchen Menge, dass die/das natürliche Polymere(n) zu der wässrigen Suspension der natürlichen Cellulosefasern in einer Menge von 15-75 Gew.-%, vorzugsweise 17-60 Gew.-%, besonders bevorzugt 20-50 Gew.-% und insbesondere 23-30 Gew.-% zugegeben werden, berechnet aus dem Gewicht des Gesamttrockenfeststoffgehalts der Suspension der natürlichen Cellulosefasern.

4. Verfahren gemäß einem der Ansprüche 1 bis 3, **dadurch gekennzeichnet, dass** das Additiv zwei oder mehrere verschiedene natürliche Polymere umfassen kann.

5. Verfahren gemäß Anspruch 1, **dadurch gekennzeichnet, dass** das Additiv Stärke und/oder Carboxymethylcellulose umfasst.

6. Verfahren gemäß einem der vorstehenden Ansprüche 1 bis 5, **dadurch gekennzeichnet, dass** das Additiv entfernt wird aus der hergestellten Suspension von mikrofibrillierter Cellulose.

7. Verfahren gemäß einem der Ansprüche 1 bis 6, **gekennzeichnet durch** die Einspeisung des Gemisches von natürlichen Cellulosefasern und des Additivs in den Homogenisator oder Fluidisator bei einer Zuführkonsistenz von 1-50 Gew.-%, vorzugsweise 1-30 Gew.-%, besonders bevorzugt 2-20 Gew.-% und insbesondere 3-15 Gew.-%, berechnet als trockene Feststoffe.

8. Verfahren gemäß einem der Ansprüche 1 bis 7, **dadurch gekennzeichnet, dass** die natürlichen Cellulosefasern aus Hartholz stammen.

9. Verfahren gemäß Anspruch 8, **dadurch gekennzeichnet, dass** die natürlichen Cellulosefasern gebleicht oder ungebleicht sind und ausgewählt sind aus Birkenfasern, Eukalyptusfasern, Akazienfasern, Espenfasern, Pappelfasern, Robinienfasern, Ahornfasern oder einem Gemisch davon, vorzugsweise gebleichten Birkenfasern.

10. Verfahren gemäß einem der Ansprüche 1 bis 9, **gekennzeichnet durch** die Zugabe des Additivs zu der Suspension von natürlichen Cellulosefasern bei einer Temperatur von < 160°C, vorzugsweise < 80°C, besonders bevorzugt < 60°C und insbesondere im Bereich von 15-35°C.

11. Verfahren gemäß einem der Ansprüche 1 bis 10, **dadurch gekennzeichnet, dass** der Homogenisator oder Fluidisator unter dem Einfluss eines Druckunterschiedes betrieben wird.

12. Verfahren gemäß einem der Ansprüche 1 bis 11, **dadurch gekennzeichnet, dass** die wässrige Suspension von natürlichen Cellulosefasern frei ist von organischen Flüssigkeiten.

13. Verfahren gemäß Anspruch 1, **dadurch gekennzeichnet, dass** die erhaltene mikrofibrillierte Cellulose einen Feststoffgehalt im Bereich von 1-50 Gew.-%, vorzugsweise 1-30 Gew.-%, besonders bevorzugt 2-20 Gew.-% und insbesondere 3-15 Gew.-% aufweist, berechnet als trockene Feststoffe, und dass sie in Form eines stabilen Gels vorliegt.

## Revendications

1. Procédé de production d'une suspension de cellulose microfibrillée ayant un diamètre de 2 à 60 nm et une longueur inférieure à 500 µm, comprenant au moins les étapes suivantes :
- l'obtention d'une suspension aqueuse de fibres de cellulose naturelle,
- l'ajout à la suspension de fibres de cellulose naturelle, d'un additif, qui consiste en au moins un polymère naturel choisi parmi la carboxyméthylcellulose (CMC), la méthylcellulose, l'hydroxypropylcellulose, l'amidon, la carraghénine, la gomme de caroube, la gomme de tamarin, le chitosan, la chitine, la gomme de guar, des dérivés cellulosiques, tels que la cellulose nanofibrillée, et l'un quelconque de leurs mélanges,
- l'apport du mélange obtenu consistant en des fibres de cellulose naturelle et de l'additif directement dans un homogénéiseur ou un fluidiseur après l'ajout de l'additif à la suspension de fibres de cellulose naturelle, et
- l'obtention d'une suspension de cellulose microfibrillée.

2. Procédé selon la revendication 1, **caractérisé par** l'ajout de l'additif dans une quantité telle que le(s) polymère(s) naturel(s) est (sont) ajouté(s) à la suspension aqueuse de fibres de cellulose naturelle en quantité de 2 à 75 % en poids, de préférence 5 à 60 % en poids, de manière davantage préférée 7 à 50 % en poids, de manière encore davantage préféré 10 à 30 % en poids, calculée à partir du poids de la teneur totale en matières sèches.

3. Procédé selon la revendication 1, **caractérisé par** l'ajout de l'additif dans une quantité telle que le(s) polymère(s) naturel(s) est (sont) ajouté(s) à la suspension aqueuse de fibres de cellulose naturelle en quantité de 15 à 75 % en poids, de préférence 17 à 60 % en poids, de manière davantage préférée 20 à 50 % en poids, de manière encore davantage préféré 23 à 30 % en poids, calculée à partir du poids de la teneur totale en matières sèches de la suspension de fibres de cellulose naturelle.

4. Procédé selon l'une quelconque des revendications 1 à 3, **caractérisé en ce que** l'additif peut comprendre deux polymères naturels différents ou plus.

5. Procédé selon la revendication 1, **caractérisé en ce que** l'additif comprend de l'amidon et/ou de la carboxyméthylcellulose.

6. Procédé selon l'une quelconque des revendications 1 à 5 précédentes, **caractérisé en ce que** l'additif est éliminé de la suspension produite de cellulose microfibrillée.

7. Procédé selon l'une quelconque des revendications 1 à 6, **caractérisé par** l'apport du mélange de fibres de cellulose naturelle et de l'additif dans l'homogéniseur ou le fluidiseur à une constante d'apport de 1 à 50 % en poids, de préférence 1 à 30 % en poids, de manière davantage préférée 2 à 20 % en poids, de manière encore davantage préférée 3 à 15 % en poids, calculée en tant que matières sèches.

8. Procédé selon l'une quelconque des revendications 1 à 7, **caractérisé en ce que** les fibres de cellulose naturelle proviennent de bois dur.

9. Procédé selon la revendication 8, **caractérisé en ce que** les fibres de cellulose naturelle sont blanchies ou non blanchies et choisies parmi des fibres de bouleau, des fibres d'eucalyptus, des fibres d'acacia, des fibres de tremble, des fibres de peuplier, des fibres de robinier, des fibres d'érable ou un mélange de celles-ci, de préférence des fibres de bouleau blanchies.

10. Procédé selon l'une quelconque des revendications 1 à 9, **caractérisé par** l'ajout de l'additif à la suspension de fibres de cellulose naturelle à une température de < 160 °C, de préférence < 80 °C, de manière davantage préférée < 60 °C, de manière encore davantage préférée dans la plage de 15 à 35 °C.

11. Procédé selon l'une quelconque des revendications 1 à 10, **caractérisé en ce que** l'homogénéisation ou la fluidisation est réalisée sous l'influence d'une différence de pression.

12. Procédé selon l'une quelconque des revendications 1 à 11, **caractérisé en ce que** la suspension aqueuse de fibres de cellulose naturelle est exempte de liquides organiques.

13. Procédé selon la revendication 1, **caractérisé en ce que** la cellulose microfibrillée obtenue a une teneur en matières sèches dans la plage de 1 à 50 % en poids, de préférence 1 à 30 % en poids, de manière davantage préférée 2 à 20 % en poids, de manière encore davantage préférée 3 à 15 % en poids, calculée en tant que matières sèches, et est sous forme d'un gel stable.
